# EUROPÄISCHE PATENTANMELDUNG

(11) **EP 4 484 564 A1**
(43) Veröffentlichungstag der Anmeldung: **01.01.2025**
(21) Anmeldenummer: 23181625.7
(22) Anmeldetag: 27.06.2023
(51) Int. Cl.: C12P 3/00, C07C 6/00, C12P 5/02, C12P 39/00

(54) **VERFAHREN ZUR STEIGERUNG DER BIOGASAUSBEUTE ANAEROBER FERMENTATIONEN UND ZUSAMMENSETZUNG KURZKETTIGER, VERZWEIGTER MONOCARBONSÄUREN**

(71) Anmelder: OQ Chemicals GmbH, 40789 Monheim am Rhein (DE)
(72) Erfinder: Klabunde, Dr. Jens, 40627 Düsseldorf (DE); Sondermann, Martin, 46414 Rhede (DE); Musenbrock, Diana, 40629 Düsseldorf (DE)
(74) Vertreter: Michalski Hüttermann & Partner Patentanwälte mbB

(57) **Zusammenfassung**

Die vorliegende Erfindung betrifft ein Verfahren zur Steigerung der Biogasausbeute einer anaeroben Biomassefermentation, mindestens umfassend die Verfahrensschritte: a) Bereitstellen einer anaerob aktiven Bakterienpopulation geeignet zur Erzeugung von Biogas aus vergärbarer Biomasse und/oder nicht-biogenen Reststoffen und b) Ein- oder mehrmalige Zugabe der vergärbaren Biomasse und/oder der nicht-biogenen Reststoffe zur Bakterienpopulation, wobei im Verfahrensschritt a), im Verfahrensschritt b) oder in beiden Verfahrensschritten zur Bakterienpopulation ein- oder mehrmals eine Mischung mindestens zweier unterschiedlicher C3-C5 Monocarbonsäuren zugegeben wird. Des Weiteren betrifft die vorliegende Erfindung eine Carbonsäurezusammensetzung mindestens umfassend drei unterschiedliche verzweigte C3-C5 Monocarbonsäuren.

## Beschreibung

Die vorliegende Erfindung betrifft ein Verfahren zur Steigerung der Biogasausbeute einer anaeroben Biomassefermentation, mindestens umfassend die Verfahrensschritte: a) Bereitstellen einer anaerob aktiven Bakterienpopulation geeignet zur Erzeugung von Biogas aus vergärbarer Biomasse und/oder nicht-biogenen Reststoffen und b) Ein- oder mehrmalige Zugabe der vergärbaren Biomasse und/oder der nicht-biogenen Reststoffe zur Bakterienpopulation, wobei im Verfahrensschritt a), im Verfahrensschritt b) oder in beiden Verfahrensschritten zur Bakterienpopulation ein- oder mehrmals eine Mischung mindestens zweier unterschiedlicher C3-C5 Monocarbonsäuren zugegeben wird. Des Weiteren betrifft die vorliegende Erfindung eine Carbonsäurezusammensetzung mindestens umfassend drei unterschiedliche verzweigte C3-C5 Monocarbonsäuren.

Die Notwendigkeit für einen schnellen und umfassenden Umbau bisheriger Energieverbrauchsgewohnheiten werden immer deutlicher. Abgesehen von den durch die Menschen verursachten, globalen Klimaveränderungen führt die Verknappung und Endlichkeit auf fossilen Kohlenstoff basierter Energieträger dazu, dass alternative Versorgungskonzepte schneller als bisher angedacht markttauglich bereitgestellt und technisch umgesetzt werden müssen. Für eine Substitution fossiler Energieträger sind technisch ausgereifte Lösungen vorhanden, welche auch heutzutage schon einen signifikanten Anteil des Energiebedarfs decken. Zu nennen sind hier beispielsweise die Windenergie und die Photovoltaik, welche in der Lage sind, in relativ großen Mengen grünen Strom bereitzustellen. Nachteiliger Weise fallen die Orte für eine geeignete Stromerzeugung und den anschließenden Stromverbrauch aber auseinander, sodass der Strom über weite Strecken transportiert werden muss. Eine dezentrale Stromerzeugung wäre wünschenswert, wirft aber noch nicht endgültig gelöste Fragen nach geeigneten Standortbedingungen und der Zwischenspeicherung überschüssiger Energie auf. Alternativ zur grünen Stromerzeugung ist eine direkte Substitution fossilen Erdgases über den Einsatz von Biogas in Form von Methan möglich und bekannt. Wie schon die Produktbezeichnungen nahelegen, weist diese Art der Substitution den Vorteil auf, dass dieselbe chemische Substanz, nur halt aus nachwachsenden biogenen Rohstoffen, bereitgestellt wird. Zudem kann aus Biogas abgeschiedenes Methan als Synthesebaustein der chemischen Industrie für vielfältige Einsatzzwecke genutzt werden. Diese sogenannte stoffliche Nutzung für die Bereitstellung nachhaltig produzierter Chemieprodukte wie Kunstdünger oder Kunststoffe ist ein essenzieller Baustein der Umgestaltung der Industrie hin zu einer nachhaltigeren Wirtschaftsweise.

Durch die Verfügbarkeit der Rohstoffquellen zu akzeptablen Kosten sowie die Weiternutzung bekannter Gas-Infrastrukturen, wie beispielsweise Brenner, Speicher und/oder Leitungsnetze, können Biogaskonzepte einen wichtigen Beitrag zur Transformation der bisherigen Energie- und Chemie-Wirtschaft liefern. Wünschenswert wäre jedoch, dass die Bereitstellungskosten für das Biogas durch eine Steigerung der Effizienz in der Erzeugung reduziert werden könnten. Ein Nachteil der nach dem Stand der Technik eingesetzten Verfahren zur Gewinnung von Biogas ist die unvollständige Umsetzung des fermentierbaren Kohlenstoffes in Kohlendioxid und Methan ("Biogas"). Verfahren zur Steigerung der Biogasausbeute sind demnach eine hoch nachgefragte Technologie, um die Bereitstellung nachhaltig erzeugter Energie und Materialien zu verbessern.

Auch in der Patentliteratur finden sich die unterschiedlichsten Ansätze zur Steigerung der Biogasproduktion.

So beschreibt beispielsweise die EP1 828 065 B1 die Verwendung eines thermophilen, anaeroben, acetogenen, Wasserstoff produzierenden Bakteriums zur Erhöhung der Methanproduktion in einem Biogas produzierenden System, wobei Biomasse unter anaeroben und thermophilen Bedingungen durch eine Polymer abbauende Mikroorganismen, acetogene Mikroorganismen und methanogene Mikroorganismen umfassende Mikroorganismengemeinschaft fermentiert wird.

In der CN 1 167 792 C wird ein bioaktiver Zusatzstoff für die Biogasfermentation offenbart. Der Zusatzstoff zeichnet sich dadurch aus, dass 100 g aktiver Zusatzstoff pro Kubikmeter Futterflüssigkeit bereitgestellt werden, wobei die Formel für den bioaktiven Zusatzstoff von 100 g Biogasfermentation lautet: Verzuckerungsenzym 40-60 g, Amylase 20-45 g, Cellulase 5∼15g, Protease 3∼10g.

In der DE102008055490A1 ist weiterhin ein Verfahren zur Erzeugung von Biogas aus Biomasse in einem Fermentierungsreaktor beschrieben, wobei der Biomasse ein Mikroorganismus der Art Clostridium sartagoformum zugesetzt wird.

Derartige aus dem Stand der Technik bekannte Lösungen können noch weiteres Verbesserungspotential bieten, insbesondere hinsichtlich der Einfachheit, der Reproduzierbarkeit und Steuerbarkeit der Steigerung in der Biogasproduktion.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung ein Verfahren sowie eine Zusammensetzung bereitzustellen, welche einfach anzuwenden ist und schon in geringen Mengen zu einer signifikanten Steigerung der Biogasproduktion in anaeroben Fermentationen führt.

Die Lösung der Aufgabe erfolgt durch die Merkmale der jeweiligen unabhängigen Ansprüche, gerichtet auf das erfindungsgemäße Verfahren sowie die erfindungsgemäße Zusammensetzung. Bevorzugte Ausgestaltungen der Erfindung sind in den Unteransprüchen, in der Beschreibung oder den Figuren angegeben, wobei weitere in den Unteransprüchen oder in der Beschreibung oder den Figuren beschriebene oder gezeigte Merkmale einzeln oder in einer beliebigen Kombination einen Gegenstand der Erfindung darstellen können, solange sich aus dem Kontext nicht eindeutig das Gegenteil ergibt.

Erfindungsgemäß ist ein Verfahren zur Steigerung der Biogasausbeute einer anaeroben Biomassefermentation, wobei das Verfahren mindestens die Verfahrensschritte umfasst:
a) Bereitstellen einer anaerob aktiven Bakterienpopulation geeignet zur Erzeugung von Biogas aus vergärbarer Biomasse und/oder nicht-biogenen Reststoffen;
b) Ein- oder mehrmalige Zugabe der vergärbaren Biomasse und/oder der nicht-biogenen Reststoffe zur Bakterienpopulation;
wobei im Verfahrensschritt a), im Verfahrensschritt b) oder in beiden Verfahrensschritten zur Bakterienpopulation ein- oder mehrmals eine Mischung mindestens zweier unterschiedlicher C3-C5 Monocarbonsäuren zugegeben wird.

Überraschenderweise wurde gefunden, dass die Zugabe kurzkettiger Monocarbonsäuren in starker Verdünnung zu einer Fermentation von Biomasse dazu führt, dass die Biogasproduktion insgesamt und insbesondere die Methanausbeute deutlich gesteigert werden kann. Die Unterschiede in der Biogasmenge ergeben sich dabei im Vergleich zu Standard-Biogasfermentationen, welche nicht die erfindungsgemäße Supplementierung mit kurzkettigen Monocarbonsäuren erhalten. Die Zugabemengen an kurzkettigen Carbonsäuren sind dabei so gering, als dass die Steigerung der Biogas- und insbesondere der Methanproduktion auf eine Verstoffwechslung der Carbonsäuren selbst zurückzuführen sein könnte. Eine Steigerung der Methanausbeute ergibt sich auch in den Fällen, in denen die für die Umsetzung der Biomasse verantwortlichen anaerob aktiven Bakterien schon ohne Zugabe von Biomasse mittels kurzkettiger Carbonsäuren vorkonditioniert werden. Ohne durch die Theorie gebunden zu sein, wird davon ausgegangen, dass die Zugabe der kurzkettigen Monocarbonsäuren einen direkten Einfluss auf die Physiologie, Syntheseleistung und auf das Zusammensetzungsverhältnis der umsetzenden Bakterienpopulation ausübt. Dabei kann nicht gänzlich ausgeschlossen werden, dass zu einem Teil in der Mikrobiologie bekannte Selektions- und Gegenselektionsprozesse eine Rolle spielen. Es wird also in biologischer und systemischer aber nicht in chemischer oder chemisch-katalytischer Sicht auf das Fermentationssystem eingewirkt. Sekundäre Effekte, wie beispielsweise eine pH-Wert-Anpassung der Fermentationsumgebung oder metabolische Effekte in der Bakterienpopulation sind sehr unwahrscheinlich, da die kurzkettigen Monocarbonsäuren nur in hohen Verdünnungen eingesetzt werden. Der Effekt ergibt sich auf jeden Fall sowohl unter der Zugabe vor der Umsetzung fermentierbarer Biomasse als auch unter einer gleichzeitigen Zugabe mit fermentierbarer Biomasse zur anaerob aktiven Bakterienpopulation.

Das erfindungsgemäße Verfahren ist ein Verfahren zur Steigerung der Biogasausbeute einer anaeroben Biomassefermentation. Die Steigerung der Biogasausbeute kann mit den üblicherweise durchgeführten Biomasse-Fermentationsverfahren kombiniert werden. Durch die Einwirkung der Carbonsäure-Zugabe auf die umsetzenden Mikroorganismen selbst, ist das Verfahren sehr unabhängig von der eingesetzten Biomasse und den konkret eingestellten Fermentationsbedingungen. Es ist allerdings wichtig, dass die Fermentationsbedingungen anaerob sind, das heißt, dass die Fermentation weitestgehend unter Luftabschluss stattfindet. Als Biogase können im Rahmen einer Biomasse-Fermentation unterschiedliche Gase entstehen. Zu diesen zählen beispielsweise Kohlendioxid, Stickstoff, Sauerstoff, Wasserstoff, Schwefelwasserstoff und Ammoniak, wobei unter Biogas hier bevorzugt Methan verstanden wird. Eine Steigerung der Gasausbeute ergibt sich in den Fällen, in denen eine zusätzliche Gasmenge durch biologische Prozesse erhalten wird, wobei der zusätzliche Gasbeitrag höher als derjenige Beitrag ist, welcher sich durch eine theoretische Verstoffwechselung der zugegebenen Monocarbonsäuren ergibt. Die Carbonsäuren werden erfindungsgemäß nicht als Substrat der Umsetzung, sondern als Modulator der Umsetzungskinetik und -Effizienz der Bakterienpopulation verstanden.

Das Verfahren umfasst den Verfahrensschritt a), in welchem das Bereitstellen einer anaerob aktiven Bakterienpopulation erfolgt, wobei die Bakterienpopulation zur Erzeugung von Biogas aus vergärbarer Biomasse und/oder nicht-biogenen Reststoffen geeignet ist. Die Grundlage der Fermentation ist das Vorliegen einer aktiven Bakterienkultur oder -Population, welche in der Lage ist, komplexe Biomasse tierischen oder pflanzlichen Ursprungs zu chemisch kleineren Bestandteilen aufzuspalten. Die anaerob aktiven Bakterien können also sämtliche Umsetzungen von Biomasse als solche bereitstellen. Zu diesen Umsetzungen gehören in der ersten Stufe des Abbaus hydrolytische Eigenschaften. Es können also längerkettige Grundsubstanzen in kürzere Bruchstücke, wie Einfachzucker, Glyzerin, Fettsäuren und Aminosäuren, zerlegt werden. Die Population ist als solche auch in der Lage die zweite Phase der Umsetzung, die Versäuerung, durchzuführen. Es werden die in der ersten Stufe gebildeten Bruchstück in Folgeprodukte, wie beispielsweise Fettsäuren, Alkohole, Wasserstoff oder Kohlendioxid umgesetzt. Die Bakterienpopulation ist auch in der Lage, die Acetogenese der umgesetzten Grundstoffe durchzuführen. In dieser Phase werden von den anaeroben Bakterien hauptsächlich Essigsäure, Wasserstoff und CO₂ gebildet. Auf Basis dieser Umsetzungsprodukte, Wasserstoff und Kohlendioxid wird schließlich Biogas, hauptsächlich in Form von Methan, durch strikt anaerobe Bakterien gebildet. Neben den üblicherweise bekannten Biomasse-Substraten können natürlich auch andere Kohlenstoffquellen, beispielsweise aus einem industriellen Umfeld, verwendet werden. Dies ist in den Fällen möglich, in denen die Bakterienpopulation prinzipiell zu Zersetzung dieser industriellen Abfall- oder Reststoffe geeignet ist. Geeignete nicht-biogene Reststoffe sind beispielsweise fermentierbare zellulosehaltige Abfälle der Papierindustrie, Klärschlamm aus industriellen oder kommunalen Abwasserreinigungsanlagen und fermentierbare Abfallströme der chemischen Industrie. Im Verfahrensschritt a) wird und wurde noch keine Biomasse aktiv zur Bakterienkultur gegeben. Die anaerobe Bakterienpopulation kann beispielsweise in Form einer Kultur als solche oder mit weiteren Substanzen vermischt oder vermengt, beispielsweise in Form eines Schlamms, vorliegen. Weitere Substanzen, neben den anaeroben Bakterien, können beispielsweise Wasser oder Sand oder Erde sein. Die weiteren Zusatzstoffe können Beiträge zur Umsetzung liefern oder sich bezüglich der Biogassynthese inert verhalten.

Das Verfahren umfasst den Verfahrensschritt b), in welchem eine ein- oder mehrmalige Zugabe der vergärbaren Biomasse und/oder der nicht-biogenen Reststoffe zur Bakterienpopulation erfolgt. Zu der Bakterienpopulation wird zum Erhalt einer batch- oder einer kontinuierlichen Biogasproduktion pflanzliche, tierische oder sonstig fermentiere Biomasse gegeben. Je nach Auslegung der Fermentation kann die Zugabe der gesamten Biomasse einmalig erfolgen. Es ist aber auch möglich, dass die Biomasse aufgeteilt zu unterschiedlichen Zeitpunkten zur Bakterienpopulation gegeben wird. Die Zeiträume der Zugabe können sich beispielsweise nach der aktuellen Umsetzung und Biogasproduktion richten. Pflanzliche Biomasse ist der biologisch abbaubare Teil von Erzeugnissen, Abfällen und Reststoffen der Land- oder Forstwirtschaft und damit verbundener Wirtschaftszweige. Tierische Biomasse besteht oder enthält nicht lebende Bestandteile (Biomasse) tierischen Ursprungs, wie beispielsweise abgestorbene oder abgestoßene Körperteile, Tierkörper und Abfälle aus Schlachtbetrieben. Dazu zählen beispielsweise ausgefallene oder ausgerissene Haare, Federn oder Schuppen. Ebenso Exuvien, Puppenhüllen, Kokonreste, Eierschalen, Eihäute-Reste und Exkremente. Zur tierischen Biomasse zählen beispielsweise auch anfallende Ausscheidungen oder Reste von Fischen aus der Fischerei, der Aquakultur und Abfällen aus der Insektenzucht. Die Zugabe der Biomasse erfolgt durch Einbringen, Ein- oder Unterrühren oder generell einem Vermengen der Biomasse und der Bakterienpopulation.

Im Verfahrensschritt a), im Verfahrensschritt b) oder in beiden Verfahrensschritten des erfindungsgemäßen Verfahrens wird zur Bakterienpopulation ein- oder mehrmals eine Mischung mindestens zweier unterschiedlicher C3-C5 Monocarbonsäuren zugegeben. Die Konditionierung der Bakterienkultur mittels Zugabe von Monocarbonsäuren kann also vor der Zugabe von Biomasse, während der Zugabe der Biomasse oder in beiden Verfahrensschritten erfolgen. Es werden dann aktiv zur Bakterienkultur oder mittelbar durch Zugabe zur Biomasse geringe Mengen an verzweigten oder geradkettigen Monocarbonsäuren zugesetzt. Die Zugabe der Monocarbonsäuren zur Bakterienpopulation kann durch Zugabe der Monocarbonsäuren als solche oder beispielsweise in Wasser gelöster Form erfolgen. Eine weitere Zugabeform der reinen oder in Wasser gelösten kurzkettigen Monocarbonsäuren ist die vorherige Aufbringung auf feste inerte Trägersubstanzen, welches die Handhabung vereinfachen kann. Die Monocarbonsäuren können einmalig oder auf mehrere Zugabemengen aufgeteilt zu der Bakterienpopulation gegeben werden. Die wiederholte Zugabe kann in regelmäßigen oder unregelmäßigen Zeitabständen erfolgen. Eine wiederholte Zugabe kann für gewisse Zeiträume unterbrochen werden. Die Monocarbonsäuren weisen eine Carbonsäuregruppe und eine gesamt C-Anzahl von 3 bis 5 auf. Die Zugabe mindestens zweier unterschiedlicher Monocarbonsäuren führt dabei zu besseren Ergebnissen als die Zugabe nur einer Monocarbonsäure. Neben den mindestens zwei Monocarbonsäuren können noch weitere Monocarbonsäuren oder andere chemische Bestandteile zur Fermentation gegeben werden. Falls die Monocarbonsäuren vor der eigentlichen Umsetzung von Biomasse zur Konditionierung der Bakterienpopulation zugegeben werden, kann die auf die Trockensubstanzmasse des Belebtschlammes bezogenen Menge beispielweise größer oder gleich 0,0001 Gew.-% und kleiner oder gleich 1 Gew.-%, weiter bevorzugt größer oder gleich 0,0002 Gew.-% und kleiner oder gleich 0,2 Gew.-% und besonders bevorzugt größer oder gleich 0,001 Gew.-% und kleiner oder gleich 0,01 Gew.-% betragen. Diese Angaben beziehen sich also auf das Gewicht der reinen Trockensubstanz des eingesetzten Belebtschlammes.

In einer bevorzugten Ausführungsform des Verfahrens können die Monocarbonsäuren verzweigte Carbonsäuren sein. Insbesondere die Zugabe kurzkettiger, verzweigter Monocarbonsäuren kann zu einer Verbesserung der Biogas- und insbesondere der Methangasausbeute in der Biomasse-Fermentation führen.

Innerhalb einer weiter bevorzugten Ausgestaltung des Verfahrens können die mindestens zwei Monocarbonsäuren aus der Gruppe bestehend aus 2-Methylbuttersäure, 3-Methylbuttersäure und Iso-Buttersäure ausgewählt sein. Insbesondere die Zugabe zweier unterschiedlicher Isomeren der Buttersäure-Monocarbonsäuren und Valeriansäuren kann zu einer Verbesserung der Biogas- und insbesondere der Methangasausbeute in der Biomasse-Fermentation führen.

Innerhalb eines weiter bevorzugten Aspektes des Verfahrens kann die Zugabe der Carbonsäuremischung im Verfahrensschritt a) erfolgen. Die Konditionierung der anaeroben Bakterienkultur kann bevorzugt auch vor dem Start der eigentlichen Biogasproduktion, also vor der Zugabe von Biomasse, erfolgen. Die Mischung der Monocarbonsäuren wird in diesem Fall also nur vor der Zugabe von Biomasse zugesetzt. Man erhält ein sehr kontrolliert und steuerbares Grundsystem, welches auch mit einem zeitlichen Abstand der Zugabe von Biomasse die Fähigkeit behält, in größeren Mengen Biogas in Form von Methan zu liefern.

Nach einer bevorzugten Charakteristik des Verfahrens kann die Biomassefermentation eine kontinuierliche Biomassefermentation sein und die Zugabe der Carbonsäuremischung zumindest zu einem Teil zusammen mit der Biomasse im Verfahrensschritt b) erfolgen. Eine kontinuierliche Biomassefermentation ergibt sich in den Fällen, in denen Biomasse über einen längeren Zeitraum, beispielsweise mehrere Wochen oder Monate, zu einer laufenden Fermentation gegeben wird. Die Carbonsäuremischung kann dabei gleichzeitig oder zeitlich verzögert zur Bakterienpopulation gegeben werden. Es ist aber auch möglich, dass die Carbonsäuremischung mit der Biomasse vermengt zur Fermentation gegeben wird. Diese Zugabe kann langanhaltend eine Steigerung der Biogasproduktion bewirken.

Nach einer weiteren bevorzugten Charakteristik des Verfahrens kann die Biomassefermentation eine kontinuierliche Biomassefermentation sein und die Zugabe der Monocarbonsäuremischung zusammen mit der Biomasse in einer kontinuierlichen Biomassefermentation im Verfahrensschritt b) erfolgen und beispielsweise nach mehreren Wochen oder Monaten die Zugabe der Carbonsäuremischung unterbrochen oder gestoppt werden.

In einer bevorzugten Ausführungsform des Verfahrens kann die Zugabe der Monocarbonsäuremischung im Verfahrensschritt b) erfolgen, wobei die Zugabe der Monocarbonsäure nicht unmittelbar mit der Zugabe der Biomasse erfolgt. Eine Zugabe der Monocarbonsäuren innerhalb eines relativ kurzen Zeitintervalls nach oder vor der Zugabe der Biomasse hat sich als besonders vorteilhaft herausgestellt. Dies gilt insbesondere im Rahmen einer kontinuierlichen Fermentation.

In einer weiter bevorzugten Ausführungsform des Verfahrens kann die Zugabe der Carbonsäuremischung in einem Zeitintervall von größer oder gleich täglich und kleiner oder gleich wöchentlich erfolgen. Eine Zugabe der Monocarbonsäuren innerhalb eines relativ kleinen Zeitintervalls, hat sich als besonders vorteilhaft herausgestellt. Dies gilt insbesondere im Rahmen einer kontinuierlichen Fermentation.

Innerhalb eines bevorzugten Aspektes des Verfahrens kann das Gewichtsverhältnis zwischen insgesamt in den Verfahrensschritten a) und/oder b) zugegebener Carbonsäuremischung und Trockensubstanzbiomasse größer oder gleich 1:1.000.000 und kleiner oder gleich 1:100 bezogen auf das im Verfahrensschritt b) eingesetzte Biomassegewicht sein. Die Zugabe dieser Monocarbonsäuremengen zur Fermentation hat sich zur Steigerung der Methangasausbeute innerhalb der Fermentation als sehr effektiv erwiesen. Die Menge an produziertem Methan kann durch diese Monocarbonsäuremengen deutlich gesteigert werden. Die Zugabemengen werden auf das Gewicht der Biomasse bezogen. Es zählt dabei das zugegebene Gesamtgewicht ohne den darin enthaltenen Wasseranteil. Im Falle der Zugabe einer feuchten Biomasse, beispielsweise in Form von Grasschnitt, wird die Zugabemenge also auf das trockene Gewicht der Biomasse ohne den Wasseranteil berechnet. Im Fall der Zugabe getrockneter Biomasse, wird nur das Gewicht der zugegebenen Menge berücksichtigt. Anorganische Bestandteile in der Biomasse bleiben unberücksichtigt. Im Rahmen einer fortlaufenden, kontinuierlichen Fermentation, mit einer theoretisch unendlichen Biomasse-Zugabemenge, wird die Zugabemenge der Biomasse innerhalb eines einmonatigen Zugabeintervalls betrachtet und die Zugabemenge an Monocarbonsäuren entsprecht berechnet. Bevorzugt kann das Gewichtsverhältnis zwischen insgesamt in den Verfahrensschritten a) und/oder b) zugegebener Carbonsäuremischung und Trockensubstanzbiomasse größer oder gleich 1:1.000.000 und kleiner oder gleich 1:100, des Weiteren bevorzugt größer oder gleich 1:500.000 und kleiner oder gleich 1:5.00 und weiterhin bevorzugt größer oder gleich 1:100.000 und kleiner oder gleich 1:1.000 betragen. Das Verhältnis ist dabei so zu verstehen, dass an der Untergrenze pro 100.000 Gewichtsteilen trockener Biomasse mindestens ein Teil Monocarbonsäuren eingesetzt werden muss. An der Obergrenze muss pro 100 Teilen trockener Biomasse weniger als 1 Teil Monocarbonsäuren eingesetzt werden. Der Trockenmassegehalt der Biomasse oder anderer Reststoffe kann beispielsweise über DIN EN 15934:2012 - Verfahren A erfolgen.

Innerhalb eines bevorzugten Aspektes des Verfahrens kann das Gewichtsverhältnis zwischen insgesamt in den Verfahrensschritten a) und/oder b) zugegebener Monocarbonsäuremischung und Trockengewicht der Bakterienpopulation, berechnet nach Trockengewicht Bakterienpopulation zu Gewicht zugegebene Monocarbonsäuren, größer oder gleich 1:100 und kleiner oder gleich 1:1.000.000 bezogen auf das Trockengewicht der im Verfahrensschritt a) vorliegenden Bakterienpopulation sein. Innerhalb dieser Monocarbonsäure-Zugabemengen ergeben sich bei relativ geringen zusätzlichen Kosten deutliche Steigerungen in der Biogas-Ausbeute. Bevorzugt kann das Verhältnis, größer oder gleich 1:200 und kleiner oder gleich 1:500.000, weiter bevorzugt, größer oder gleich 1:1000 und kleiner oder gleich 1:100.000 betragen. Das Trockengewicht der Bakterien kann ebenfalls über die DIN EN 15934:2012 - Verfahren A bestimmt werden, wobei das Trockengewicht dann noch anorganische Substanzen umfasst.

In einer weiter bevorzugten Ausführungsform des Verfahrens kann jede der mindestens zwei Monocarbonsäuren in der Mischung einen Gewichtsanteil von größer oder gleich 20 Gew.-% und kleiner oder gleich 70 Gew.-% aufweisen, wobei die Gewichtsanteile sich zu 100 Gew.-% ergänzen. Insbesondere Monocarbonsäure-Mischungen mit einem signifikanten Mengenanteil jeweils unterschiedlicher Monocarbonsäuren können zur Verbesserung der Methanausbeute anaerober Fermentation beitragen. Bevorzugt kann jede der mindestens Monocarbonsäuren in der Mischung einen Gewichtsanteil von größer oder gleich 25 Gew.-% und kleiner oder gleich 60 Gew.-% aufweisen, des Weiteren bevorzugt einen Gewichtsanteil von größer oder gleich 30 Gew.-% und kleiner oder gleich 50 Gew.-%. Die Gewichtsanteile beziehen sich auf die gesamt zugegebene Menge an erfindungsgemäßen Monocarbonsäuren, wobei dann die Menge an Monocarbonsäuren sich zu 100 Gew.-% ergänzen. Die Mischung kann ebenfalls weitere Substanzen aufweisen, wobei diese weiteren Bestandteile nicht zum Gewichtsverhältnis der Monocarbonsäuren beitragen.

Im Rahmen einer weiterhin bevorzugten Ausgestaltung des Verfahrens kann jede der mindestens zwei Monocarbonsäuren in der Mischung einen Gewichtsanteil von größer oder gleich 25 Gew.-% und kleiner oder gleich 50 Gew.-% aufweisen, wobei die Gewichtsanteile sich zu 100 Gew.-% ergänzen. Insbesondere Monocarbonsäure-Mischungen mit einem signifikanten Mengenanteil jeweils unterschiedlicher Monocarbonsäuren können zur Verbesserung der Methanausbeute in anaeroben Fermentationen beitragen. Bevorzugt kann jede der mindestens Monocarbonsäuren in der Mischung einen Gewichtsanteil von größer oder gleich 30 Gew.-% und kleiner oder gleich 42 Gew.-% aufweisen, des Weiteren bevorzugt kann der Gewichtsanteil größer oder gleich 31 Gew.-% und kleiner oder gleich 38 Gew.-% betragen. Die Gewichtsanteile beziehen sich auf die gesamt zugegebene Menge an Monocarbonsäuren der entsprechenden Größe.

In einer weiteren Ausgestaltung des Verfahrens kann die Monocarbonsäuremischung mindestens drei unterschiedliche Monocarbonsäuren ausgesucht aus der Gruppe der C3-C5 Monocarbonsäuren aufweisen, wobei jede der drei Monocarbonsäuren in der Mischung einen Gewichtsanteil von größer oder gleich 30 Gew.-% und kleiner oder gleich 40 Gew.-% aufweist, wobei die Gewichtsanteile sich zu 100 Gew.-% ergänzen. Die Zugabe von mindestens drei unterschiedlichen Monocarbonsäuren in annähernd gleichen Mengen und ohne größere Mengen weiterer Monocarbonsäure-Zusätze scheint für die Steigerung der Methangas-Ausbeute in anaeroben Fermentationen besonders geeignet. Die Methanausbeute wird signifikant gesteigert und die zusätzlichen Kosten für die Zugabe der Monocarbonsäuren aufgrund der nur geringen Einsatzmengen klein gehalten. Auf diese Art und Weise lässt sich die Wirtschaftlichkeit des Gesamtverfahrens erhöhen.

In einer bevorzugten Ausführungsform des Verfahrens kann die Biomasse eine Grüngut-Biomasse sein. Gerade bei der Umsetzung von Biomasse pflanzlichen Ursprung kann die Fermentationsleitung der Bakterienpopulation durch die Zugabe der Monocarbonsäuremischung deutlich gesteigert werden. Es wird signifikant mehr Methan produziert, welches die Wirtschaftlichkeit des Gesamtverfahrens verbessert.

Innerhalb einer weiter bevorzugten Ausgestaltung des Verfahrens kann das Grüngut aus der Gruppe bestehend aus Bambus, Zuckerrohr, Gras, Klee, Leguminosen, Luzerne, Weidegras oder Mischungen daraus ausgesucht sein, wobei das Grüngut als Frischware, Silage oder als Mischung aus Frischware und Silage zur Fermentation gegeben werden kann. Die Umsetzung von Grüngut aus der oben angegebenen Gruppe zu Biogas kann durch die Zugabe von Monocarbonsäuren deutlich verbessert werden. Es wird signifikant mehr Methan produziert, welches die Wirtschaftlichkeit des Gesamtverfahrens verbessert.

In einer bevorzugten Ausführungsform kann die Biomasse Getreide umfassen oder aus Getreide bestehen. Das Getreide kann beispielsweise aus der Gruppe bestehend aus Mais, Weizen, Roggen, Reis, Hirse, Gerste, Hafer, Triticale und Dinkel oder Mischungen daraus ausgesucht sein. Das Getreide kann als Frischware, Silage oder als Mischung aus Frischware und Silage zur Fermentation gegeben werden. Die Zugabe der erfindungsgemäßen Monocarbonsäuremischung kann in der Verstoffwechselung dieser Biomasse zu besonders hohen Steigerungen der Methanausbeute führen.

In einer bevorzugten Ausführungsform kann die Biomasse aus tierischer Biomasse ausgesucht sein. Die tierische Biomasse kann beispielsweise aus der Gruppe aus abgestorbenen oder abgestoßenen Körperteilen, Tierkörper und Abfälle aus Schlachtbetrieben, ausgefallene oder ausgerissene Haare, Federn oder Schuppen, Exuvien, Puppenhüllen, Kokonreste, Eierschalen, Eihäute-Reste, Exkremente, Ausscheidungen oder Reste von Fischen aus der Fischerei, und der Aquakultur und Ausscheidungen und Reste aus der Insektenzucht bestehen. Die Zugabe der erfindungsgemäßen Monocarbonsäuremischung kann in der Verstoffwechselung dieser Biomasse zu besonders hohen Steigerungen der Methanausbeute führen.

Innerhalb eines weiter bevorzugten Aspektes des Verfahrens kann das Biogas Methan sein. Durch die Zugabe einer Mischung unterschiedlicher, kurzkettiger Monocarbonsäuren kann die Syntheseleistung und Zusammensetzung der anaeroben Bakterienpopulation in Bezug auf Methan deutlich verbessert werden.

Des Weiteren erfindungsgemäß ist eine Monocarbonsäurezusammensetzung mindestens umfassend drei unterschiedliche verzweigte C3-C5 Monocarbonsäuren, wobei der Gewichtsanteil jeder einzelner dieser Monocarbonsäuren in der Mischung größer oder gleich 25 Gew.-% und kleiner oder gleich 40 Gew.-% beträgt. Eine Monocarbonsäurezusammensetzung mit oben angegebenen Mengenanteilen an verzweigten C3-C5 Monocarbonsäuren kann als Supplement in anaeroben Fermentationen dazu beitragen, dass die Syntheseleistung bezogen auf das Wertprodukt Methan deutlich verbessert wird. Die Steigerung der Syntheseleistung ergibt sich dabei über eine physiologische Wirkung der Mischung auf die Bakterienpopulation und nicht durch die Verstoffwechselung der Monocarbonsäuren selbst. Bevorzugt kann die Zusammensetzung einen Gewichtsanteil jeder einzelner dieser Monocarbonsäuren in der Mischung von größer oder gleich 27,5 Gew.-% und kleiner oder gleich 37,5 Gew.-% und weiter bevorzugt von größer oder gleich 30,0 Gew.-% und kleiner oder gleich 35,0 Gew.-% aufweisen. Die Gesamt-Gewichtsanteile der erfindungsgemäßen Monocarbonsäuren ergänzen sich dabei zu 100 Gew.-%. Es können aber in der Mischung noch weitere Bestandteile vorliegen. Dies können beispielsweise Hilfsstoffe, Lösemittel oder sonstige Bestandteile sein, deren Gewicht aber nicht zur Berechnung der Gewichtsverhältnisse der erfindungsgemäß einsetzbaren Monocarbonsäuren herangezogen wird. Besonders bevorzugt kann die Mischung aus den erfindungsgemäßen Monocarbonsäuren bestehen.

In einer weiter bevorzugten Ausführungsform der Monocarbonsäurezusammensetzung kann die Carbonsäuremischung 2-Methylbuttersäure, 3-Methylbuttersäure und Iso-Buttersäure umfassen. Insbesondere Mischungen aus den oben angegebenen Monocarbonsäuren können einen besonders positiven Effekt auf die Methanausbeute in einer Biogasfermentation ausüben. Insbesondere kann die Methanproduktion deutlich gesteigert werden. Bevorzugt kann Zusammensetzung zu größer oder gleich 90 Gew.-% aus diesen Monocarbonsäuren bestehen. Bevorzugt kann das Gewichtsverhältnis der drei Monocarbonsäuren in der Mischung 1:1:1 betragen. Besonders bevorzugt kann die Mischung aus diesen drei Monocarbonsäuren bestehen.

Weitere Vorteile und vorteilhafte Ausgestaltungen der erfindungsgemäßen Gegenstände werden durch die Figuren veranschaulicht und in den nachfolgenden Beispielen erläutert. Dabei ist zu beachten, dass die Figuren nur beschreibenden Charakter haben und nicht dazu gedacht sind, die Erfindung in irgendeiner Form einzuschränken.

### Beispiele

### A) Statischer Gärtest mit Grassilage

Der Einfluss einer kurzkettigen Monocarbonsäure-Mischung auf die Biogassynthese-Leistung einer anaeroben Fermentation wurde mittels eines Gärtest durchgeführt. Als Monocarbonsäure-Mischung wurde eine 1:1:1-(Gewichts)-Mischung der drei Monocarbonsäuren 2-Methylbuttersäure, 3-Methylbuttersäure und Iso-Buttersäure verwendet. Als Biomasse kam Grassilage zum Einsatz. Die Grassilage wies folgende Charakteristiken auf: Trockensubstanzmassegehalt 29,8 % OS (nach DIN EN 15934:2012), einen Glühverlust von 87,7 % TS (nach DIN EN 15935:2021) und eine organische Trockensubstanz von 26,3 %. Die Monocarbonsäuren wurden zur Vorkonditionierung der Bakterienpopulation eingesetzt. Die Zugabe erfolgte im Verfahrensschritt a), also vor der Zugabe von Biomasse. Die Zugabe der Monocarbonsäuren erfolgte aufgeteilt, durch regelmäßige Zugabe der Säuremischung über einen Zeitraum von 4 Wochen bei einer Inkubationstemperatur von 40°C. Parallel wurde eine Bakterienpopulation unter gleichen Bedingungen aber ohne Zugabe des Additivs als Kontrolle mitgeführt. Im Anschluss wurden jeweils 30g Grassilage mit 500g der konditionierten Impfschlämme sowie mit 500g des Kontrollansatzes gemischt und diese Mischung anschließend mit Wasser auf ein Gesamtvolumen von 1000 ml aufgefüllt. Die Impfschlämme wiesen einen Trockensubstanzgehalt von ca. 4% sowie einen Glühverlust von ca. 71 % auf. Die Gemische wurden in gasdicht verschließbare Reaktionsgefäße mit Magnetventil gesteuerten Gasauslässen überführt. Die Inkubation der Ansätze erfolgte in einem auf 40°C (mesophil) temperiertem Wasserbad. Die Aufnahme der Gasproduktion erfolgte durch einen Trommelgaszähler. Die Bestimmung des Methangehaltes im Biogas wurde mittels eines Methan-IR-Sensor durchgeführt. Pro Versuch wurden jeweils 3 unabhängige Ansätze gefahren. Die Kontrolle (Impfschlamm ohne weitere Zugabe von Monocarbonsäuren) wurde zweifach ausgeführt.

Die Zugaben der erfindungsgemäßen Monocarbonsäure-Mischung wie oben angegeben sowie Wasser für die Kontrollen ergab sich wie folgt:

| Tag | 1:2.500 | 1:10.000 | 1:50.000 | Kontrolle |
|---|---|---|---|---|
| 0 | 0,04 | 0,010 | 0,002 | 0,04 |
| 1 | 0,06 | 0,015 | 0,003 | 0,05 |
| 9 | 0,06 | 0,015 | 0,003 | 0,06 |
| 11 | 0,04 | 0,010 | 0,002 | 0,04 |
| 14 | 0,04 | 0,010 | 0,002 | 0,04 |
| 17 | 0,04 | 0,010 | 0,002 | 0,04 |
| 21 | 0,04 | 0,010 | 0,002 | 0,04 |

Die Zugabemengen sind in g angegeben und beziehen sich auf jeweils 1 I Impfschlamm. Die Angaben in der Kopfzeile beziehen sich auf die Gewichts-Zugabemengen an Monocarbonsäuren im Verhältnis zum Gewicht der später zugesetzten Biomasse.

Die Gärtests nach Zugabe der Biomasse wurden bei einer Temperatur von 40°C durchgeführt. Es wurden jeweils 500 g Impfschlamm und 30 g Substrat vergärt. Es ergeben sich folgende Gärresultate:

| | 1:2.500 | 1:10.000 | 1:50.000 | Kontrolle |
|---|---|---|---|---|
| Impfschlamm oTS in g | 14,7 | 14,4 | 14,4 | 14,2 |
| Grassilage oTS in g | 7,86 | 7,86 | 7,86 | 7,86 |
| Summe oTS in g | 22,56 | 22,26 | 22,25 | 22,06 |
| Biogasausbeute | 434,0 | 436,4 | 423,5 | 379,0 |
| in NI/kg oTS | (+/- 11,9) | (+/- 5,4) | (+/- 7,4) | (+/- 11,5) |
| Methankonzentration Vol-% | 53,2 | 53,4 | 53,8 | 54,9 |
| Methanausbeute NI/ g oTS | 231,0 (+/- 5,7) | 233,1 (+/- 2,2) | 227,9 (+/- 4,0) | 208,2 (+/- 5,9) |

| | | | | |
|---|---|---|---|---|
| oTS = organische Trockensubstanz, NI = Normliter | | | | |

In sämtlichen Ansätzen setzte die Gasproduktion unmittelbar nach dem Start der Untersuchung ein. Eine ausgeprägte lag-Phase ohne Gasproduktion konnte nicht beobachtet werden. Die Ansätze mit den konditionierten Bakterienpopulationen zeigen im Vergleich zum Kontrollansatz von Beginn an eine höhere Ausgasungsgeschwindigkeit. Nach 10 Tagen Inkubationsdauer liegen die Ansätze mit 1:2.500 und 1:10.000 in Bezug auf den spezifischen Methanertrag jeweils um ca. 19 % oberhalb des Kontrollansatzes. Der Mehrertrag des Ansatzes 1:50.000 liegt mit rund 15 % leicht unterhalb der beiden ersten Ansätze mit höheren Monocarbonsäuren-Zugabemengen. Im weiteren Verlauf reduzieren sich die Mehrerträge der Ansätze mit den konditionierten Impfschlämmen zwar, blieben aber bis zum Ende des Versuches signifikant oberhalb der des Kontrollansatzes.

Zum Endpunkt der Untersuchung nach 30 Tagen weist der Ansatz 1:2.500 einen spezifischen Methanertrag von 231,0 Nl/kg oTS auf und liegt damit ca. 11 % oberhalb der Leistung des Kontrollansatzes (208,2 Nl/kg oTS). Der Ansatz 1:10.000 erreicht 233,2 Nl/kg oTS, welches einem Mehrertrag von rund 12 % entspricht. Mit 222,9 Nl/kg oTS und ca. 9,5 % Mehrertrag liegt der Ansatz mit einer Zugabemenge von 1:50.000 weiterhin leicht unterhalb der Ansätze mit Zugaben von 1:2500 und 1:10.000. Allerdings liegen die Unterschiede zwischen den konditionierten Ansätzen mit < 3% innerhalb der versuchsbedingten Abweichungen und sind nicht als signifikant anzusehen. Die prozentualen Mehrerträge in Bezug auf den gesamten Bioertrag liegen mit 14,5 % (1:2.500), 15,1 % (1:10.000) und 11,7 % (1:50:000) leicht oberhalb der jeweiligen Methan-Mehrerträge. Dies ist auf den höheren Methangehalt des Kontrollansatzes zurückzuführen.

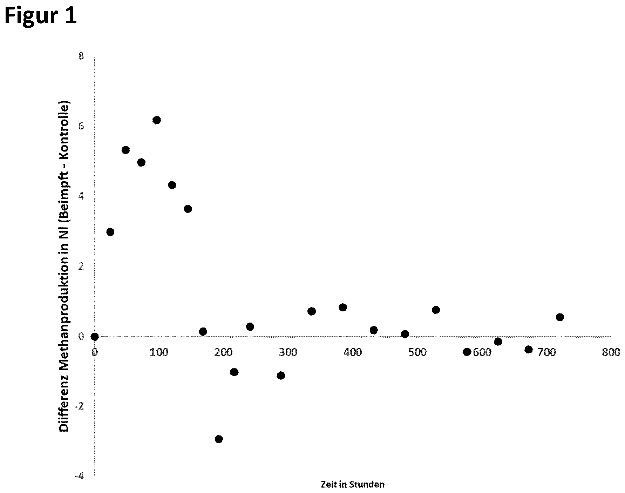

In der Figur 1 ist der Unterschied in der Methanproduktion zwischen der Kontrolle und dem Versuchsansatz mit einer Zugabemenge von 1:10.000 dargestellt. Es ist deutlich zu sehen, dass die Methanproduktion besonders am Anfang der Umsetzung gesteigert wird. Der Effekt lässt im Lauf der Zeit nach, aber auch nach sehr langen Zeiträumen führt die Vorkonditionierung der Bakterienpopulation zu einer verbesserten Methanausbeute. Die verbesserte Methanausbeute lässt sich aufgrund der Größenordnung nicht auf die Verstoffwechselung der Monocarbonsäuren zurückführen. Die verbesserte Ausbeute muss sich aufgrund einer physiologischen Konditionierung der Bakterien, mit anschließend verbesserter Umsetzung der Biomasse ergeben.

### B) Statischer Gärtest mit Hühnertrockenkot (HTK)

Der Einfluss einer kurzkettigen Monocarbonsäure-Mischung auf die Biogassynthese-Leistung einer anaeroben Fermentation wurde mittels eines Gärtest gemäß Beispiel A) durchgeführt. Anstelle der Grassilage kam aber in diesem Versuch als Biomasse Hühnertrockenkot (HTK) zum Einsatz. Es ergeben sich vergleichbare Resultate. Durch Zugabe geringer Mengen einer erfindungsgemäßen Monocarbonsäuremischung kann die Biogas-Syntheseleistung und insbesondere die Methan-Syntheseleistung auch bei der Verwendung von HTK als Biomasse signifikant gesteigert werden.

### C) Statischer Gärtest mit Maissilage

Der Einfluss einer kurzkettigen Monocarbonsäure-Mischung auf die Biogassynthese-Leistung einer anaeroben Fermentation wurde mittels eines Gärtest gemäß Beispiel A) durchgeführt. Anstelle der Grassilage kam aber in diesem Versuch als Biomasse Maissilage zum Einsatz. Es ergeben sich vergleichbare Resultate. Durch Zugabe geringer Mengen einer erfindungsgemäßen Monocarbonsäuremischung kann die Biogas-Syntheseleistung und insbesondere die Methan-Syntheseleistung auch bei der Verwendung von Maissilage als Biomasse signifikant gesteigert werden.

### Beispiel D) Kontinuierlicher Gärtest Grassilage

Der Einfluss einer erfindungsgemäßen kurzkettigen Monocarbonsäure-Mischung auf die Biogassynthese-Leistung einer anaeroben Fermentation wurde mittels eines kontinuierlichen Gärtests durchgeführt. Als Monocarbonsäure-Mischung wurde eine 1:1:1-(Gewichts)-Mischung von drei Monocarbonsäuren 2-Methylbuttersäure, 3-Methylbuttersäure und Iso-Buttersäure verwendet. Als Biomasse kam Grassilage zum Einsatz. Die Monocarbonsäuren wurden zur Vorkonditionierung der Bakterienpopulation eingesetzt. Die Zugabe erfolgte im Verfahrensschritt a) kombiniert mit Verfahrensschritt b), also vor und während der Zugabe von Biomasse. Die Zugabe der Monocarbonsäuren erfolgte aufgeteilt, durch tägliche Zugabe der Säuremischung über einen Zeitraum von 21 Tagen bei einer Inkubationstemperatur von 40°C in einem durchmischten Reaktor mit 70 Liter Arbeitsvolumen. Es wurden täglich in zwei parallel konditionierten Gärtests 7 mL der Monocarbonsäuremischung hinzugegeben und eine Bakterienpopulation unter gleichen Bedingungen aber ohne Zugabe des Additivs als Kontrolle mitgeführt. Die Impfschlämme wiesen einen organischen Trockensubstanzgehalt von ca. 8% auf. Im Anschluss wurden jeweils 250 g Trockensubtanzmasse Grassilage täglich zu den 70 Liter konditionierten Impfschlämme hinzugegeben. In einer Variante wurden 0,5 mL Monocarbonsäuremischung im Mischungsverhältnis 1:500 bezogen auf die zugegebene organische Trockensubstanzmasse Grassilage und in einer weiteren Variante 0,25 mL Monocarbonsäuremischung im Mischungsverhältnis 1:1000 bezogen auf die zugegebene organische Trockensubstanzmasse Grassilage zu den konditionierten Gärtests hinzugegeben. Die Aufnahme der Gasproduktion erfolgte durch einen Trommelgaszähler. Eine Variante mit täglicher Zugabe von 0,5 mL der Monocarbonsäuremischung zeigte nach 13 Tagen täglicher Zugabe von 250 Gramm Trockensubstanzmasse Grassilage eine tägliche Mehrbildung von Biogas von ungefähr 10% bezogen auf die Gesamtgasmenge an Biogas in der Kontrolle der keine Monocarbonsäuremischung hinzugefügt wurde. Hiermit wurde gezeigt, dass auch die Syntheseleistung einer kontinuierlichen Fermentation deutlich verbessert werden kann. Auch wurde gezeigt, dass die Zugabe der Monocarbonsäuren begleitend zur Zugabe der Biomasse erfolgen kann.

## Patentansprüche

1. Verfahren zur Steigerung der Biogasausbeute einer anaeroben Biomassefermentation, mindestens umfassend die Verfahrensschritte:
a) Bereitstellen einer anaerob aktiven Bakterienpopulation geeignet zur Erzeugung von Biogas aus vergärbarer Biomasse und/oder nicht-biogenen Reststoffen;
b) Ein- oder mehrmalige Zugabe der vergärbaren Biomasse und/oder der nicht-biogenen Reststoffe zur Bakterienpopulation;
**dadurch gekennzeichnet, dass** im Verfahrensschritt a), im Verfahrensschritt b) oder in beiden Verfahrensschritten zur Bakterienpopulation ein- oder mehrmals eine Mischung mindestens zweier unterschiedlicher C3-C5 Monocarbonsäuren zugegeben wird.

2. Verfahren nach Anspruch 1, wobei die Monocarbonsäuren verzweigte Monocarbonsäuren sind.

3. Verfahren nach einem der vorherigen Ansprüche, wobei die mindestens zwei Monocarbonsäuren ausgewählt sind aus der Gruppe bestehend aus 2-Methylbuttersäure, 3-Methylbuttersäure und Iso-Buttersäure.

4. Verfahren nach einem der vorherigen Ansprüche, wobei die Zugabe der Monocarbonsäuremischung im Verfahrensschritt a) erfolgt.

5. Verfahren nach einem der Ansprüche 1 bis 3, wobei die Biomassefermentation eine kontinuierliche Biomassefermentation ist und die Zugabe der Monocarbonsäuremischung, zumindest zu einem Teil, zusammen mit der Biomasse im Verfahrensschritt b) erfolgt.

6. Verfahren nach Anspruch 5, wobei die Zugabe der Monocarbonsäuremischung in einem Zeitintervall von größer oder gleich täglich und kleiner oder gleich wöchentlich erfolgt.

7. Verfahren nach einem der vorherigen Ansprüche, wobei das Gewichtsverhältnis zwischen insgesamt in den Verfahrensschritten a) und/oder b) zugegebener Carbonsäuremischung und Trockensubstanzbiomasse größer oder gleich 1:1.000.000 und kleiner oder gleich 1:100 bezogen auf das im Verfahrensschritt b) eingesetzte Biomassegewicht ist.

8. Verfahren nach einem der vorherigen Ansprüche, wobei jede der mindestens zwei Monocarbonsäuren in der Monocarbonsäuremischung einen Gewichtsanteil von größer oder gleich 20 Gew.-% und kleiner oder gleich 70 Gew.-% aufweist, wobei die Gewichtsanteile der Monocarbonsäuren in der Monocarbonsäuremischung sich zu 100 Gew.-% ergänzen.

9. Verfahren nach einem der vorherigen Ansprüche, wobei jede der mindestens zwei Monocarbonsäuren in der Monocarbonsäuremischung einen Gewichtsanteil von größer oder gleich 25 Gew.-% und kleiner oder gleich 50 Gew.-% aufweist, wobei die Gewichtsanteile der Monocarbonsäuren in der Monocarbonsäuremischung sich zu 100 Gew.-% ergänzen.

10. Verfahren nach einem der vorherigen Ansprüche, wobei die Monocarbonsäuremischung mindestens drei unterschiedliche Monocarbonsäuren ausgesucht aus der Gruppe der C3-C5 Monocarbonsäuren aufweist, wobei jede der drei Monocarbonsäuren in der Mischung einen Gewichtsanteil von größer oder gleich 30 Gew.-% und kleiner oder gleich 40 Gew.-% aufweist, wobei die Gewichtsanteile der Monocarbonsäuren in der Monocarbonsäuremischung sich zu 100 Gew.-% ergänzen.

11. Verfahren nach einem der vorherigen Ansprüche, wobei die Biomasse eine Grüngut-Biomasse ist.

12. Verfahren nach Anspruch 11, wobei das Grüngut ausgesucht ist aus der Gruppe bestehend aus Bambus, Zuckerrohr, Gras, Klee, Leguminosen, Luzerne, Weidegras oder Mischungen daraus, wobei das Grüngut als Frischware, Silage oder als Mischung aus Frischware und Silage zur Fermentation gegeben werden kann.

13. Verfahren nach einem der vorherigen Ansprüche, wobei das Biogas Methan ist.

14. Monocarbonsäurezusammensetzung mindestens umfassend drei unterschiedliche verzweigte C3-C5 Monocarbonsäuren, wobei der Gewichtsanteil jeder einzelner dieser Monocarbonsäuren in der Mischung größer oder gleich 25 Gew.-% und kleiner oder gleich 40 Gew.-% beträgt.

15. Monocarbonsäurezusammensetzung nach Anspruch 14, wobei die Monocarbonsäuremischung zu größer oder gleich 90 Gew.-% aus den Monocarbonsäuren 2-Methylbuttersäure, 3-Methylbuttersäure und Iso-Buttersäure besteht.
